# EUROPEAN PATENT SPECIFICATION

(11) **EP 4 209 186 B1**
(45) Date of publication and mention of the grant of the patent: **11.12.2024**
(21) Application number: 22217169.6
(22) Date of filing: 29.12.2022
(51) Int. Cl.: A61B 17/29, A61B 18/14, A61B 17/28

(54) **LAPAROSCOPIC INSTRUMENT**
LAPAROSKOPISCHES INSTRUMENT
INSTRUMENT LAPAROSCOPIQUE

(30) Priority: 07.01.2022 PL 44009522
(43) Date of publication of application: 12.07.2023
(73) Proprietor: "Konmex" Spólka Z Ograniczona Odpowiedzialnoscia, 00-716 Warsaw (PL)
(72) Inventor: Wawryniuk, Grzegorz, 02-495 Warsaw (PL); Korczak-Cegielska, Ilona, 03-890 Warsaw (PL); Brodaczewski, Wieslaw, Dublin, K32WY89 (IE); Gajda, Pawel, 21-345 Borki (PL)
(74) Representative: Kondrat, Mariusz

(56) References cited:
- EP-A1- 3 841 991
- US-A- 5 893 835

## Description

The object of the invention is the laparoscopic instrument.

In surgical operations performed on internal organs using the laparoscopic method, various types of surgical instruments are used to hold, deflect and incise the tissue of the operated organs or organs connected to the operated organs with the possibility of simultaneous coagulation. Changing the position of the actuators, for example, the pliers, is realized by changing the position of the lever relative to the handgrip. Commonly used are laparoscopic instruments in which the lever can occupy any position relative to the handgrip to grasp or release the tissue. Similarly, in a scissor-type instrument, the lever can occupy any position relative to the handgrip. Very often, a laparoscopic instrument is equipped with a knob for changing the angular position of the actuators, which makes it easier to operate with difficult access to tissues. There is a demand that the knob allows any angular positioning of the working plane of the actuators.

Equally common are instruments equipped with pliers for holding tissue coupled to a mechanism for holding the jaws of the pliers in a fixed reciprocal operative position providing a specific compression of the tissue, which must remain temporarily maintained due to the course of the operation. The operative position is also understood as holding the tissue to allow temporary deflection or clamping of the tissue. Activities such as grasping, tissue incision can be performed repeatedly during the operation according to the course of the operation, so the design of the laparoscopic instrument should be ergonomic. There is an expectation, that the handling of the laparoscopic instrument should be as simple as possible, and the movement resistance of the mechanisms during the handling of the instrument should be as low as possible.

From the state of the art laparoscopic instruments are known, which are equipped with a handgrip, wherein the handgrip can be two-part such as in scissors, as for which the tightening of the parts of the handgrip against each other results in the handling of the instrument's actuators. Instruments having a handgrip on which a lever is attached for handling the actuators are known. The handgrips of the instruments are often equipped with mechanisms for maintaining a temporarily unchanging position of the lever.

Document EP2675371B1 discloses a laparoscopic instrument comprising a shank with attached actuators, a handgrip and a lever drawn against the handle. The instrument can be used in various positions by the person performing the laparoscopic procedure. The instrument is equipped with a knob for rotating the shank together with the actuators, but the knob is not equipped with a mechanism for locking the shank in the desired angular position.

Document EP0598607B1 discloses a laparoscopic instrument having a handgrip to which a handle is tightened for manipulating the pliers. The instrument has a knob for rotating the shank and actuators. The instrument is equipped with a latching mechanism including an arched toothed bar integrated in the handle and a latch located on the handgrip, wherein the latch encompasses a tooth on the longitudinal support. A deflection lever is installed on the handgrip, which is adapted to occupy two positions, wherein the lever maintains continuous contact with the longitudinal support. In one position of the deflecting lever, the toothed bar remains interlocked with the tooth, and in the other position it deforms the longitudinal support so that the tooth is moved away and out of the interlock so that the latching mechanism does not operate. The disclosed latching mechanism is constructed of multiple components, and furthermore, the performance of the latching mechanism may be impaired with the loss of elastic properties of the components from which the mechanism is constructed.

Document US5293878A discloses a laparoscopic instrument that is equipped with a latching mechanism having two ball-shaped latching elements that alternate the angular position of the shank and the actuators, wherein the recesses for the latching elements are made at the proximal end of the shank. The latching mechanisms operate transverse to the axis of the shank. In addition, the laparoscopic instrument is equipped with a latching mechanism comprising a tooth mounted on a prone component and a tooth bar that engages with this tooth.

The European application EP3841991A1 discloses a laparoscopic instrument comprising a handgrip and an articulated lever, a shank attached to the handgrip, tissue acting actuators installed at the further end of the shank, a string to move the actuators connected to the actuators and to the lever. The instrument is equipped with an integrated latching mechanism, comprising a toothed bar and a latch, designed to hold the actuators in the operating position by holding the lever position in relation to the handgrip. Moreover the instrument is provided with a locking and releasing mechanism equipped with a switching lever to hold the latching mechanism to engage and to disengage the latching mechanism, the switching lever being rotatably mounted on the handgrip.

Document US5893835A discloses an ultrasonic surgical clamp coagulator apparatus configured to effect cutting, coagulation, and clamping of tissue by cooperation of a clamping mechanism of the apparatus with an associated ultrasonic end-effector. In the housing there is provided a clutch mechanism for rotational positioning of an elongated portion and end-effector. The arrangement permits the elongated portion to be rotatably positioned with respect to the apparatus housing and permits rotation of the end-effector relative to the elongated portion.

The essence of the invention is a laparoscopic instrument comprising a handgrip; a lever pivotally attached to the handgrip; a shank embedded in a sleeve pivotally attached to the handgrip, wherein the proximal end of the shank is embedded in the sleeve, and wherein the distal end of the shank has attached actuators for acting on the tissue; a knob mounted on the sleeve for rotating the sleeve along with the shank; a latching mechanism for determining the angular position of the sleeve and shank relative to the handgrip; a string for moving the actuators, with the distal end of the string being connected to said actuators and the proximal end of the string being connected to a lever, wherein the string is located inside the shank and the movement of the lever is coupled to the movement of the actuators. The laparoscopic instrument is characterized in that the latching mechanism is located at the tip of the handgrip opposite the lever, wherein the tip is equipped with latching slots and the inner part of the knob adjacent to the sleeve is equipped with a latching element and a spring.

The instrument is characterized in that the sleeve has a flange and a groove, and the cylindrically shaped inner part of the knob has slits separating the inner part into segments, wherein at least one segment has an abutment facing the axis of the shank, wherein at least one abutment is inserted into the groove for determining the position of the knob with respect to the sleeve, while the flange is brought to a retaining surface in the handgrip.

The instrument is characterized in that the latch sockets are made on the front surface of the handgrip tip, and the latching element carries out a movement parallel to the axis of the shank during the movement of the knob.

The instrument is characterized in that the shank is equipped with a recess in the proximal part of the shank, while the sleeve is equipped with an internal projection that is inserted into the recess of the shank for determining the position of the sleeve relative to the shank.

Advantageously, the sleeve has a slit separating the part of the sleeve opposite the flange into two parts.

The instrument is characterized in that the proximal end of the string has a narrowing and is connected to the lever by means of a joint comprising a cylindrical element having a slotted hole, in which an insert having a notch against which the narrowing of the string is located, in addition, the cylindrical element has a transverse hole in which the proximal end of the string is located.

Advantageously, the laparoscopic instrument is equipped with a latching mechanism comprising an arched toothed bar connected to a lever and a latching tooth connected to a handgrip, adapted to hold the position of the actuators in an operative position by holding the position of the lever relative to the handgrip, and a locking and releasing mechanism equipped with a switch lever for holding the latching mechanism in gear and for releasing the latching mechanism, wherein the arched toothed bar is pivotable, and the latching tooth is located stationary on the handgrip, wherein the switching lever, fixed pivotally on the handgrip, is equipped with a pushback element for deflecting the arched toothed bar, which pushback element in the release position of the switching lever triggers the latching mechanism in such a way, that the arched toothed bar is offset from the latching tooth, wherein the pushback element rests against the arched toothed bar of the latching mechanism, while in the locking position the pushback element is offset from the arched toothed bar of the latching mechanism.

Advantageously, the switching lever is fastened with pivots to snap into the first opening in the handgrip.

Advantageously, the lever is equipped with pivots that are adapted for mounting in the second opening in the handgrip.

Advantageously, the lever is equipped with a third opening, which is adapted for mounting a pivot element for the string.

Advantageously, a fourth opening is located next to the third opening for mounting the cap of the third opening.

Advantageously, the pushback element is made as a pin.

The actuators can be selected from a group comprising clamp jaws, window jaws, serrated jaws, hook jaws, scissors.

The handgrip can be equipped with an electrical connector.

The latching mechanism located at the tip of the handgrip allows high resolution angular positioning of the clamping jaws, scissors or other actuators. In addition, the design of the instrument according to the invention comprising a minimum number of parts, the design and assembly are simpler, and the cost of manufacturing the instrument is reduced. Determining the angular position with the latching mechanism is performed on the maximum possible diameter available on the handgrip, so that less force is needed to maintain the fixed position, the resistance to movement of the knob resulting from the latching mechanism is reduced.

The use of a cylindrical joint with a transversely placed insert reduces the resistance to movement during rotation of the string to a very great extent compared to known instruments, the resistance to movement has been reduced to a very small value because the contact area of the insert with the constriction is only a small part of the circular cross-section of the string.

The laparoscopic instrument according to the invention enables aural control of the change in the angular position of the actuators, both intentional caused by the operator and unintentional caused by accident, which is appreciated as the specificity of endoscopic procedures limits the surgeon to observe the movement of the hands and instruments. In the instrument according to the invention, the execution of the function of rotation through the shank of the instrument is clearly perceptible and/or heard by means of a series of clicks, which allows auditory control of the rotation of the actuators. The device according to the invention is equipped with a latching mechanism that provides high angular resolution, i.e. it allows the shank to be retained together with the jaws after rotation by a very small angle.

The object of the invention is described below in relation to the illustrated embodiments shown in the drawing, in which:
Fig. 1 shows the first embodiment of the laparoscopic instrument in perspective view,
Fig. 1a shows the enlarged view of the scissors from Fig. 1,
Fig. 2 shows the second embodiment of the laparoscopic instrument in perspective view,
Fig. 2a shows the enlarged view of the jaws from Fig. 2,
Fig. 3 shows the laparoscopic instrument of Fig. 2 in exploded view,
Fig. 3a shows the enlarged view of the string joint,
Fig. 4 shows the middle part of the laparoscopic instrument with a partial detachment,
Fig. 5 shows the laparoscopic instrument of Fig. 2 in longitudinal section, wherein the locking-release mechanism is in the locking position,
Fig. 6 shows the enlarged view of the instrument section from Fig. 5,
Fig. 7 shows the laparoscopic instrument in longitudinal section, wherein the locking-release mechanism is in the release position, and
Fig. 8 shows the enlarged view of the instrument section from Fig. 7.

In the following description, the terms "proximal" and "distal" are used to refer to the elongated or curved support or string elements of the instrument, wherein the term "proximal" refers to the end of the elongated element on the side opposite the handgrip, while the term "distal" refers to the end of such element on the side opposite the handgrip.

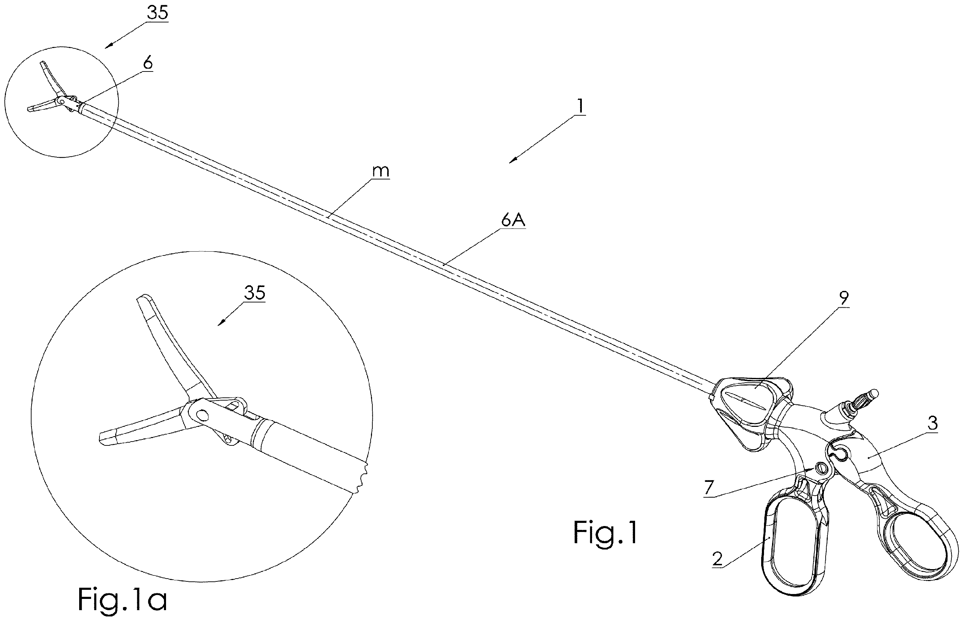

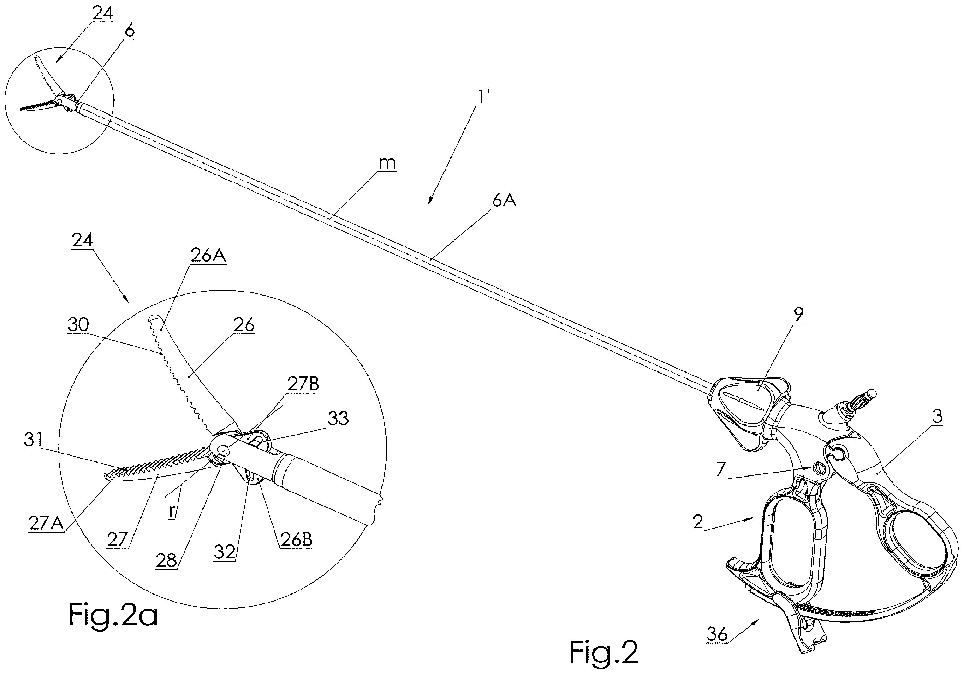

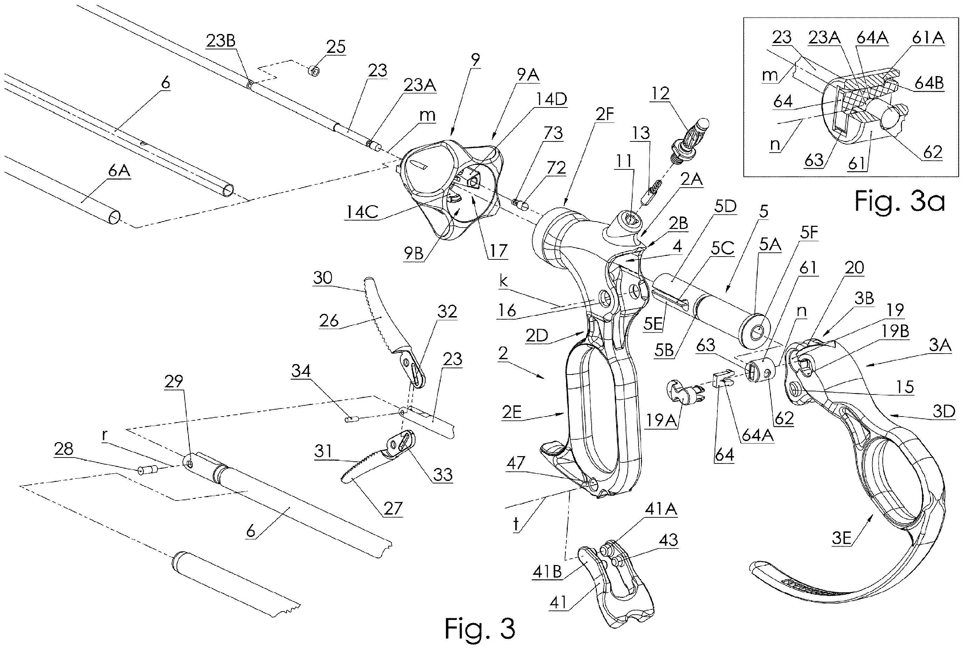

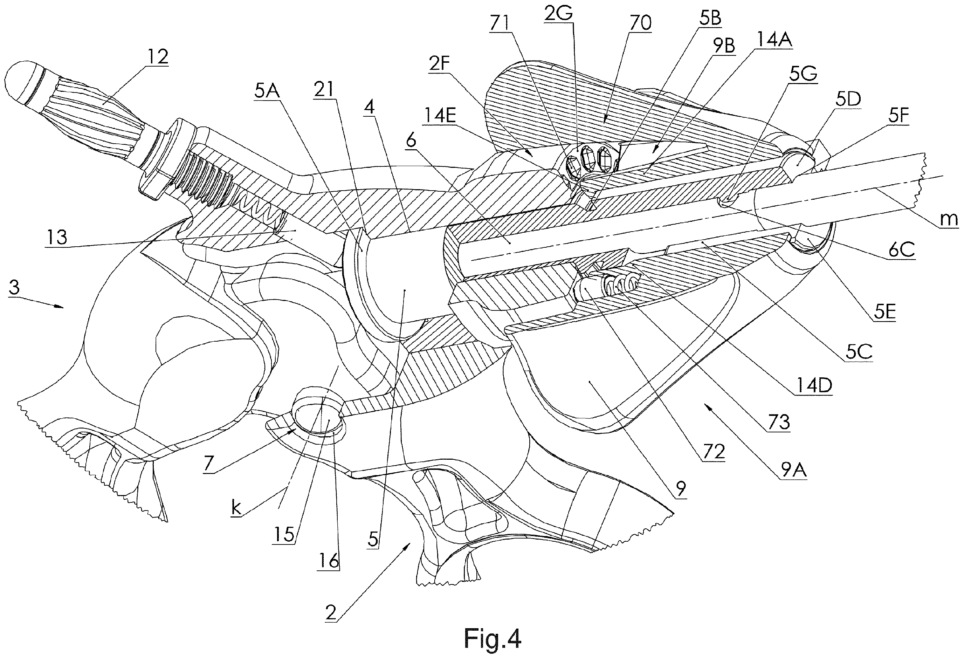

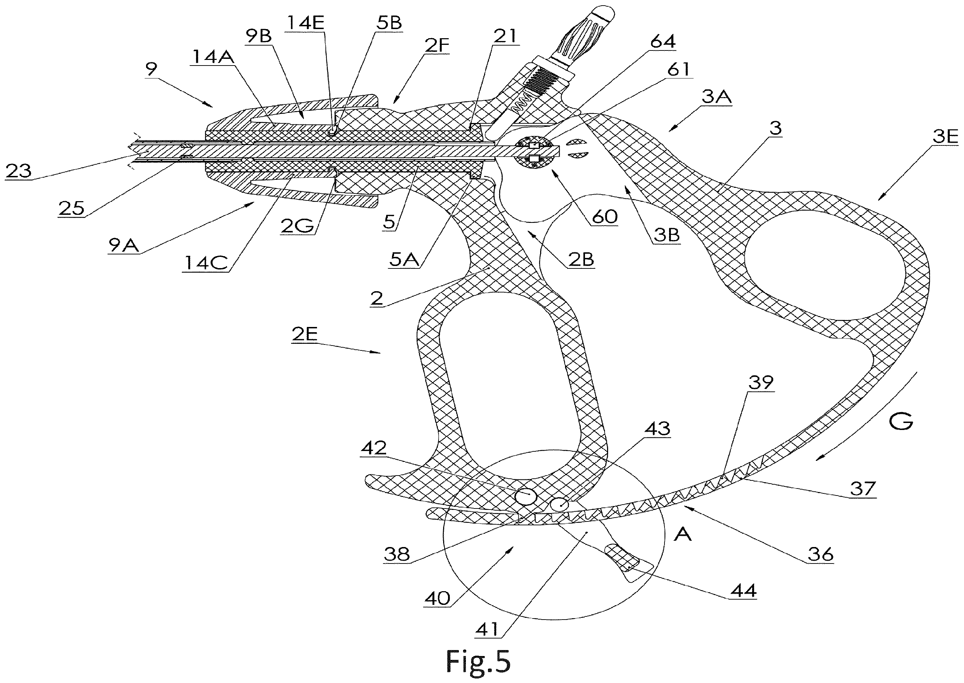

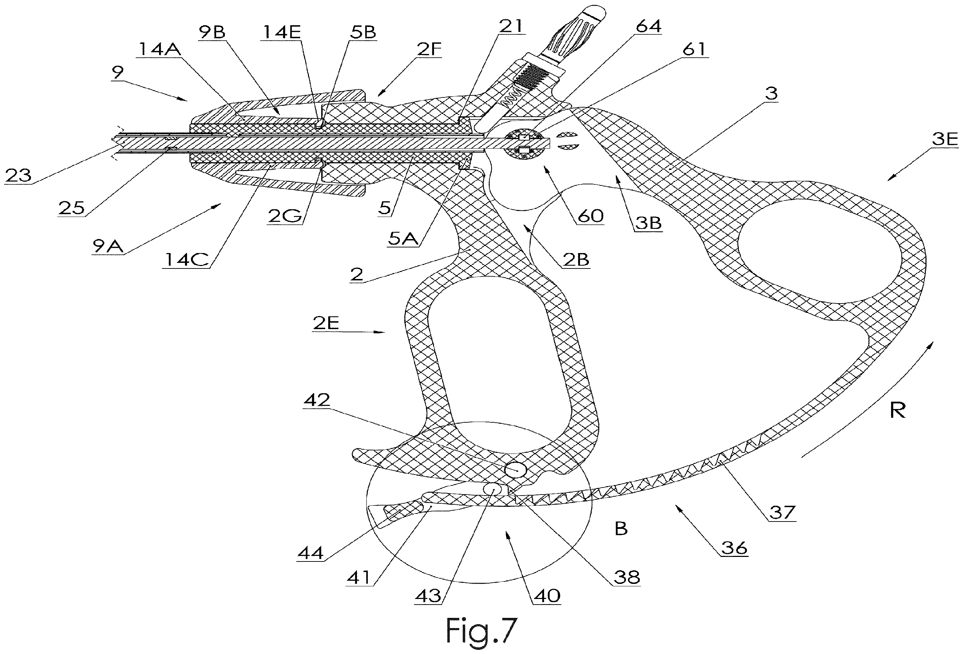

The laparoscopic instrument 1 shown in Fig. 1 in the embodiment has a handgrip 2 and a lever 3 adapted to be operated with the hand, wherein the handgrip 2 and the lever 3 are in the form of handles as for scissors, with finger openings. The lever 3 is operated with the thumb, and the handgrip 2 is gripped with the other fingers, wherein the handgrip 2 is pointed essentially downward when using the laparoscopic instrument 1. In the laparoscopic instrument shown in Fig. 1, the position of the lever 3 relative to the handgrip 2 can be varied as desired, the actuators 35 for this instrument are scissors. The laparoscopic instrument 1' shown in Fig. 2 is constructed similarly, wherein the lever 3 is provided with a latching mechanism 36 which allows the lever 3 to be locked relative to the handgrip 2, in the example of manufacture shown, the laparoscopic instrument 1' is provided with clamping jaws 24. In both embodiments, the lever 3 and the handgrip 2 are connected to each other with their upper parts 3A and 2A by means of a joint 7. In the upper part 2A of the handgrip 2 there is a cylindrical longitudinal opening 4 (Figs. 3 and 4), in which an m-axis shank 6 is fixed by means of a sleeve 5, wherein the shank 6 in the form of a tubular support passes centrally through the sleeve 5. The sleeve 5 is fixed pivotally in the handgrip 2. A knob 9 is mounted on the sleeve 5, which is equipped with an outer part 9A for gripping the knob 9 and a cylindrically shaped inner part 9B, which is adjacent to the sleeve 5. The knob 9 will be discussed later in the description. The knob 9 is used for determining the angular position of the shank 6 and thus the actuators 24, 35 of the laparoscopic instrument 1, 1' at the same time. The cylindrically shaped inner portion 9B comprising four segments 14A, 14B, 14C and 14D separated by slits 17. The slits 17 may extend parallel to the m-axis of the shank 6 and along the cylindrically shaped inner portion 9B, the slits 17 may be of any shape. The sleeve 5 has a flange 5A and a groove 5B, which serve to fix the position of the sleeve 5 in the handgrip 2 in the direction of the m-axis of the shank 6. The flange 5A is brought against the retaining surface 21, i.e., it rests against the retaining surface 21 in the handgrip 2, and the abutment 14E on the segment 14A, 14C is inserted into the groove 5B, wherein the segment 14A rests against the front surface 2G of the tip 2F of the handgrip 2. Opposite segment 14A, segment 14C (Figs. 5 and 7), i.e. the segment resting on the other side of the m-axis, is also equipped with an abutment 14E inserted into groove 5B. Segment 14C also rests against the front surface 2G of the handgrip 2 tip 2F.

The sleeve 5 has a longitudinal slit 5C separating parts 5D and 5E (Figs. 3 and 4), wherein the plane of the slit 5C rests on the axis m. Parts 5D and 5E can be symmetrical with respect to the plane passing through the axis of the sleeve 5 and can be tilted. On the wall of the opening 5F of the sleeve 5 an inner protrusion 5G is located, which is inserted into the recess 6C of the shank 6. When the inner protrusion 5G is inserted into the recess 6C, the position of the sleeve 5 with respect to the shank 6 is fixed. The recess 6C can take the form of an opening with an axis perpendicular to the m-axis.

The laparoscopic instrument is equipped with a latching mechanism 70 for determining the angular position of the shank 6 relative to the handgrip 2 (Fig. 4). The latching mechanism 70 comprises latching sockets 71 formed on the front surface 2G of the tip 2F of the handgrip 2, as well as a latching element 72 and a spring 73. The tip 2F of the handgrip 2 is located opposite to the lever 3. The latching seat 71 may be in the form of a spherical or spherical-roller recess, while the latching element 72 may be in the form of a ball or a ball-ended roller. The latching element 72 is embedded in the segment 14D of the inner portion 9B of the knob 9. The knob 9 may be provided with two latching elements 72, the second latching element 72 in an invisible segment 14B. The latching element 72 is provided with the ability to perform movement in the direction parallel to the m-axis of the handgrip 2 while rotating the knob 9 and jumping between the latching seats 71. When setting the position of the actuators 24, 35 required during the operation by rotating the knob 9, the operating personnel can hear a series of clicks of the latching mechanism 70. Regardless of the aural evaluation of the change in the position of the actuators, the operating personnel can feel the successive latches of the latching mechanism 70 with their hand. The operating personnel obtains information about the intended or accidental rotation of the shank and at the same time the actuators, which are located inside the patient during the surgery, without having to look at the handgrip of the laparoscopic instrument.

In the upper part 2A of the handgrip 2 an opening 11 is located, in which the electrical connector HF 12 is fixed over the spring latch 13. The electrical connector HF 12 presses against the spring latch 13 so that, by tensioning the spring, the latch 13 holds a frictionally conductive connection to the shank 6, while in the section from the knob 9 to the actuators of the laparoscopic instrument 1, 1', the shank 6 is enclosed by an insulating film 6A, for example, of heat-shrinkable material. The laparoscopic 1, 1' instrument equipped with an electrical connector can be used in laparoscopic surgery as a monopolar instrument. The banana-type electrical connector 12 is a standard connector used in electrosurgery for supplying current to the actuators. The connector 12 is located at a 55-degree angle to the axis of the shank 6, so that the power cord is arranged in a way that does not interfere with the movements and precision of the surgeon. The laparoscopic instrument 1, 1' is equipped with a seal 25 embedded in the groove 23B of the string 23, which is adjacent to the inner surface of the stem 6.

The lever 3 is attached to the handgrip 2 in an articulated manner on an axis k. The lever 3 is equipped in its upper part 3A with two coaxial pivots 15 located on two opposite sides of the shaded part 3B. The pivots 15 and the openings 16 form a joint 7. The shaded part 3B is located in the recess 2B of the handgrip 2, wherein the pivots 15 are placed in the second openings 16, the k-axis of which passes through the walls of the recess 2B, wherein the k-axis is below the m-axis of the shank 6 when the instrument is in use. Due to this arrangement of the m and k axes, the joint 7 of the lever 3 is below the string 23, and the lever 3 does not enter the area of the string 23.

The string 23 is connected to the lever 3 by means of a joint 60 of the string 23 located in the shaded portion 3B of the lever 3 adjacent to the wider portion 3C of the upper portion 3A of the lever 3. The joint 60 of the string 23 comprising a cylindrical element 61, an insert 64 and a proximal end of the string 23 having a narrowing 23A. The cylindrical element 61 is pivotally embedded in a cylindrical third opening 19 having an n-axis in the shaded portion 3B. The cylindrical element 61 has a transverse opening 62 made transversely to the n-axis of the cylindrical element 61. The proximal end of the string 23 is inserted into the transverse opening 62. The cylindrical element 61 is positioned in the third opening 19 symmetrically with respect to the upper portion 3A so that the n-axis is located perpendicular to the symmetry plane of the slotted cutout 20. The diameter of the transverse opening 62 is adapted to the diameter of the proximal end of the string 23. The cylindrical element 61 has a slotted opening 63 made in the base of the cylinder parallel to the n-axis of the cylindrical element 61, and an insert 64 is placed in the slotted opening 63. The insert 64 has a cutout 64A, which is applied to the narrowing 23A of the proximal end of the string 23 after the proximal end of the string 23 is inserted into the opening 62 (Fig. 3a). The insert 64 is provided with latches 64B, which enter the cutouts 61A in the cylindrical element 61. When the insert 64 is latched, the cutout 64A comprises the narrowing 23A. The cutout 64A can be V-shaped or U-shaped. The narrowing 23A is in the form of a circumferential groove the width of which is greater than the thickness of the insert 64, and the diameter of the narrowing is less than or equal to the width of the cutout 64A. The string 23 can rotate while remaining in contact with the insert 64, wherein only a small portion of the circular section of the string 23 remains in contact with the insert 64. The joint 60 of the string 23 has two degrees of freedom, one degree of freedom being imparted by the cylindrical connection of the cylindrical element 61 with the third opening 19, and the other degree of freedom being imparted by the connection of the narrowing 23A of the proximal end of the string 23 with the cutout 64A of the insert 64. The resistance to movement in rotating the actuators from friction between the insert 64 and the narrowing 23A is negligible.

In the shaded portion 3B of the upper portion 3A, a slotted cutout 20 is made, the plane of which is perpendicular to both the k-axis and the n-axis, wherein the k- and n-axes intersect the slotted cutout 20. The third opening 19 is closed by a cap 19A snapped into a fourth opening 19B located next to the third opening 19.

The laparoscopic instrument may be equipped with actuators selected from a group comprising: clamp jaws, window jaws, toothed jaws, hooked jaws, scissors, in medical terminology known as graspers, dissectors and scissors of various types. In the second embodiment shown, in the laparoscopic instrument 1', pliers 24 are used comprising two toothed clamping jaws 26, 27 fixed pivotally on the r-axis (Figs. 2a and 3) by means of a pin 28 embedded in a bracket 29 at the distal end of the shank 6. Each clamping jaw 26, 27 is made as a double-sided lever, one arm of 26A, 27A respectively has a clamping surface 30, 31, the other arm of 26B, 27B respectively is provided with an opening 32, 33 into which a pin 34 is inserted and fixed in the distal end of string 23. The pin 34 moves in the openings 32, 33, which makes the jaws move.

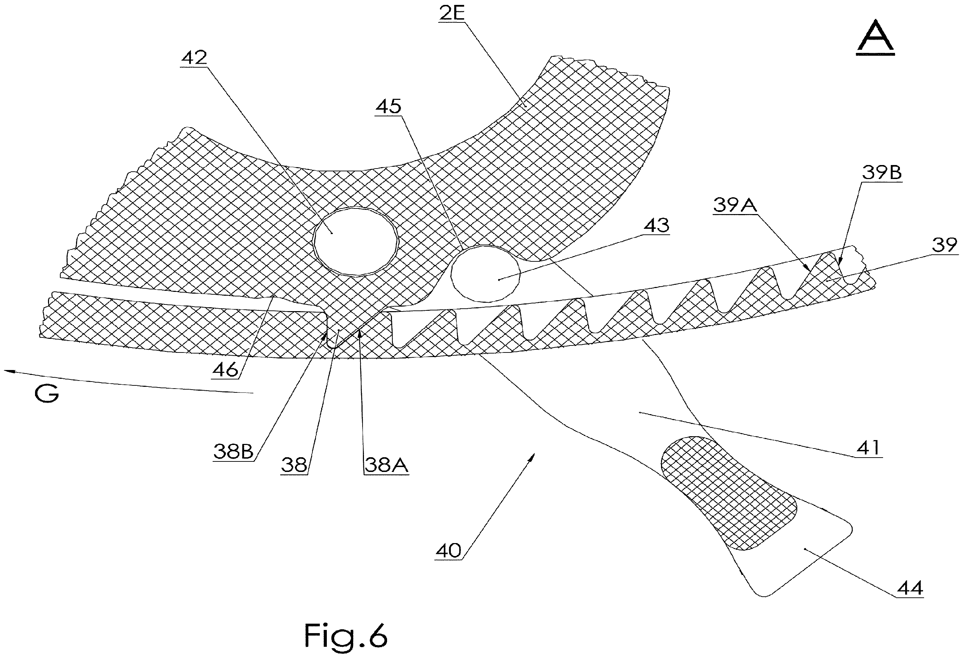

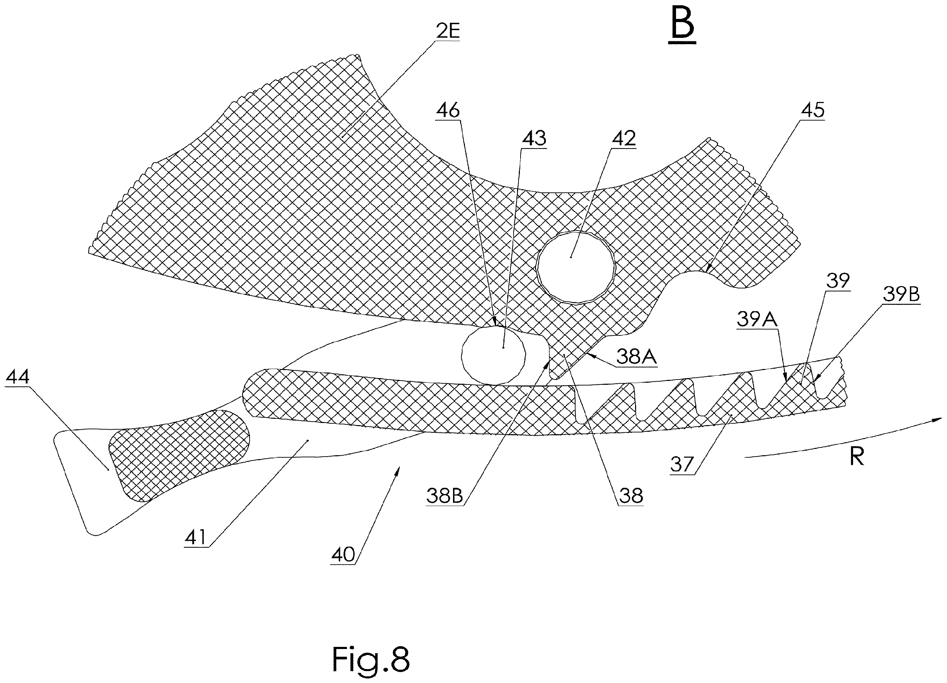

Handgrip 2 and lever 3 together form a gripping-handling device. Handgrip 2 is equipped with a first central element 2D and a first gripping element 2E, while lever 3 is equipped with a second central element 3D and a second gripping element 3E (Fig. 3). The laparoscopic instrument 1' in the second embodiment is equipped with a latching mechanism 36 comprising a toothed bar 37 and a cooperating latching tooth 38 acting as a latch (Figs. 5 to 8). The arched toothed bar 37 is integrated with the second gripping element 3E, i.e., it can be made in combination with the gripping element 3E, the toothed bar 37 is equipped with a plurality of teeth 39 adapted to cooperate with the tooth 38 located on the first gripping element 2E. The toothed bar 37 is positioned so that the teeth 39 of the toothed bar 37 come into contact with the tooth 38, i.e., they engage the tooth 38. Both the teeth 39 and the tooth 38 are triangular in shape. Between each of the teeth 39 there is a recess matching the tooth 38. During the movement marked as G that tightens the lever 3 to the handgrip 2, i.e. the second gripping element 3E to the first gripping element 2E, the teeth 39 jump over the tooth 38 i.e. successive surfaces 39A of the teeth 39 come into contact with the surface 38A of the tooth 38 (Figs. 6 and 8), while the toothed bar 37 is flexible and can be deflected to allow the teeth 39 to jump over. The movement of lever 3 in the direction of G affects the clamping of the pliers, i.e. the pressing of lever 3 against handgrip 2, and causes the jaws 26, 27 or any other actuators to move closer together. When the operator reaches the required operating position, in this case clamping i.e. the required position of the pliers 26, 27 with respect to each other and when the pulling movement of the lever 3 to the handgrip 2 is stopped, the tooth 38 remains in the recess between the teeth 39. Self-withdrawal of the lever 3 and thus self-release of the clamp is not possible because the surface 39B of the tooth 39 rests against the surface 38B of the tooth 38. The laparoscopic instrument 1' is equipped with a locking and releasing mechanism 40, which is adapted to occupy two positions - a locking position, in which it is possible to clamp the pliers 24 to any degree of clamping and retain such clamping, and a release position, in which the operating personnel can freely clamp the pliers 24 and release the clamp, but it is not possible to retain the clamping of the pliers. The locking and releasing mechanism 40 comprise a switching lever 41 articulated on the first gripping element 2E in the first opening 47 (Fig. 3), wherein the t-axis of the joint 42 is located substantially parallel to the k-axis and the n-axis. The switching lever 41 is provided with a pushback element 43, which can be positioned depending on the position of the switching lever 41 in either the first slot 45 for the locking position (Figs. 5 and 6) or the second slot 46 for the releasing position (Figs. 7 and 8). The pushback element 43 provides a movement stop to the switching lever 41, in the embodiment shown, the pushback element 43 is in the form of a cylindrical pin. Switching lever 41 is adapted to change its position from locking to releasing and vice versa by pressing tip 44. The joint 42 of the switching lever 41 comprises two pivots 41A located opposite to each other, situated on the arms 41B of the switching lever 41. The arms 41B are flexible, which allows them to be gently tilted during installation on the handgrip 2 so that the switching lever 41 is attached to the latch by inserting the pivots 41A into the first opening 47 in the handgrip 2.

In the locking position of the locking and releasing mechanism 40 as shown in Figs. 5 and 6, the switching lever 41 is in the locking position and the latching mechanism 36 works as described above, i.e., it is possible to lock the appropriate operating position of the actuators, in the embodiment shown, the clamping of the jaws 26, 27 of the pliers 24 of the laparoscopic instrument 1' by the operating personnel. In Figs. 7 and 8, the locking and releasing mechanism 40 is in the releasing position, it is possible to withdraw the lever 3 in the direction marked as R. Switching lever 41 is in the release position, where pushback element 43 is resting in the second socket 46 of gripping element 2E.

The handgrip 2, the lever 3, the cap 19A, the switching lever 41, the knob 9, the cylindrical element 61 and the insert 64 are made of plastic, for example ABS, PEEK, using the plastic injection molding technique. Handgrip 2 and toothed bar 37 may be one piece obtained by this technique greatly simplifying the tool compared to examples known in the state of the art.

## Claims

1. The laparoscopic instrument (1, 1') comprising
a handgrip (2);
a lever (3) pivotally attached to the handgrip (2);
a shank (6) embedded in a sleeve (5) pivotally attached to the handgrip (2), wherein the proximal end of the shank (6) is embedded in the sleeve (5), however, on the distal end of the shank (6) actuators (24, 35) are attached to interact with the tissue;
a knob (9) mounted on the sleeve (5) for rotating the sleeve (5) along with the shank (6); a latching mechanism (70) for determining the angular position of the sleeve (5) and shank (6) relative to the handgrip (2);
a string (23) for moving actuators (24, 35), wherein the distal end of the string (23) is connected to said actuators (24, 35), and the proximal end of the string (23) is connected to the lever (3), wherein the string (23) is located inside the shank (6), and the movement of the lever (3) is coupled to the movement of the actuators (24, 35), **characterized in that** the latching mechanism (70) is located at the tip (2F) of the handgrip (2) opposite the lever (3), wherein the tip (2F) is provided with latching seats (71), and the inner part (9B) of the knob (9) adjacent to the sleeve (5) is provided with a latching element (72) and a spring (73).

2. The instrument according to claim. 1, **characterized in that** the sleeve (5) has a flange (5A) and a groove (5B), and the cylindrically shaped inner part (9B) of the knob (9) has slits (17) separating the inner part (9B) into segments (14A, 14B, 14C, 14D), wherein at least one segment (14A, 14C) has an abutment (14E) facing the axis (m) of the shank (6), wherein at least one abutment (14E) is inserted into the groove (5B) for determining the position of the knob (9) with respect to the sleeve (5), while the flange (5A) is brought up to the retaining surface (21) in the handgrip (2).

3. The instrument according to claim 1 or 2, **characterized in that** the latching seats (71) are formed on the front surface (2G) of the tip (2F) of the handgrip (2), and the latching element (72) makes a movement in the direction parallel to the axis (m) of the shank (6) during the movement of the knob (9).

4. Instrument according to any one of claims 1 to 3, **characterized in that**, the shank (6) is provided with a recess (6C) in the proximal part of the shank (6), however, the sleeve (5) is provided with an inner protrusion (5G) which is inserted into the recess (6C) of the shank (6) for determining the position of the sleeve (5) with respect to the shank (6).

5. The instrument according to any one of claims 2 to 4, **characterized in that** the sleeve (5) has a slit (5C) separating the part of the sleeve (5) opposite to the flange (5A) into two parts (5D, 5E).

6. The instrument according to any one of claims 1 to 5, **characterized in that** the proximal end of the string (23) has a narrowing (23A) and is connected to the lever (3) by means of a joint (60) of the string (23) comprising a cylindrical element (61) having a slotted opening (63), in which an insert (64) having a notch (64A) is located, against which the narrowing (23A) of the string (23) is supported, in addition, the cylindrical element (61) has a transverse opening (62) in which the proximal end of the string (23) is located.

7. The instrument according to any one of claims 1 to 6, **characterized in that** it is equipped with a latching mechanism (36) comprising an arched toothed bar (37) connected to the lever (3) and a latching tooth (38) connected to the handgrip (2), adapted to retain the position of the actuators (24, 35) in the operational position by holding the position of the lever (3) relative to the handgrip (2), and with a locking and releasing mechanism (40) equipped with a switching lever (41) for holding the latching mechanism (36) in gear and for tripping the latching mechanism (36), wherein the arched toothed bar (37) can be pivoted, and the latching tooth (38) is positioned immovably on the handgrip (2), wherein the switching lever (41) mounted pivotally on the handgrip (2) is equipped with a pushback element (43) for deflecting the arched toothed bar (37), wherein the pushback element (43) in the release position of the switching lever (41) disengages the latching mechanism (36) in such a way, that the arched toothed bar (37) is moved away from the latching tooth (38), wherein the pushback element (43) rests against the toothed bar (37) of the latching mechanism (36), however, in the locking position the pushback element (43) is moved away from the arched toothed bar (37) of the latching mechanism (36).

8. The instrument according to claim 7, **characterized in that**, the switching lever (41) is fastened with pivots (41A) to latch into a first opening (47) in the handgrip (2).

9. The instrument according to claims 1 to 8, **characterized in that**, the lever (3) is equipped with pivots (15), which are adapted for mounting in second openings (16) in the handgrip (2).

10. The instrument according to claims 1 to 9, **characterized in that**, the lever (3) is provided with a third opening (19), which is adapted for mounting an articulating element (61) for the string (23).

11. The instrument according to claim 10, **characterized in that**, alongside the third opening (19), there is a fourth opening (19B) for fastening the cap (19A) of the third opening (19).

12. The instrument according to any one of claims 7 to 11, **characterized in that** the pushback element (43) is made as a pin.

13. The instrument according to claims 1 to 12, **characterized in that** the actuators are selected from a group comprising clamp jaws, window jaws, toothed jaws, hooked jaws, scissors.

14. The device according to claims 1 to 13, **characterized in that** the handgrip (2) is equipped with an electrical connector (12).

## Patentansprüche

1. Das laparoskopische Instrument (1, 1') umfasst
einen Haltegriff (2);
einen Hebel (3), der schwenkbar am Handgriff (2) befestigt ist;
einen Schaft (6), der in einer Hülse (5) eingebettet ist, die schwenkbar an dem Handgriff (2) angebracht ist, wobei das proximale Ende des Schafts (6) in der Hülse (5) eingebettet ist, jedoch am distalen Ende des Schafts (6) Betätigungselemente (24, 35) angebracht sind, um mit dem Gewebe zusammenzuwirken;
einen an der Hülse (5) angebrachten Knopf (9) zum Drehen der Hülse (5) zusammen mit dem Schaft (6); einen Verriegelungsmechanismus (70) zum Bestimmen der Winkelposition der Hülse (5) und des Schaftes (6) relativ zum Handgriff (2);
eine Schnur (23) zum Bewegen von Aktuatoren (24, 35), wobei das distale Ende der Schnur (23) mit den Aktuatoren (24, 35) verbunden ist und das proximale Ende der Schnur (23) mit dem
Hebel (3), wobei die Schnur (23) innerhalb des Schaftes (6) angeordnet ist und die Bewegung des Hebels (3) mit der Bewegung der Betätigungselemente (24, 35) gekoppelt ist, **dadurch gekennzeichnet, dass** der Verriegelungsmechanismus (70) an der Spitze (2F) des Handgriffs (2) gegenüber dem Hebel (3) angeordnet ist, wobei die Spitze (2F) mit Verriegelungssitzen (71) versehen ist und der innere Teil (9B) des Knaufs (9), der an die Hülse (5) angrenzt, mit einem Verriegelungselement (72) und einer Feder (73) versehen ist.

2. Das Instrument nach Anspruch. 1, **dadurch gekennzeichnet, dass** die Hülse (5) einen Flansch aufweist.
(5A) und eine Nut (5B) aufweist, und der zylindrisch geformte Innenteil (9B) des Knopfes (9) Schlitze (17) aufweist, die den Innenteil (9B) in Segmente (14A, 14B, 14C, 14D) unterteilen, wobei mindestens ein Segment (14A, 14C) ein der Achse (m) des Schaftes (6) zugewandtes Widerlager (14E) aufweist, wobei mindestens ein Widerlager (14E) in die Nut (5B) eingesetzt wird, um die Position des Knopfes (9) in Bezug auf die Hülse (5) zu bestimmen, während der Flansch (5A) an die Haltefläche (21) im Handgriff (2) herangeführt wird.

3. Instrument nach Anspruch 1 oder 2, **dadurch gekennzeichnet, dass** die Verriegelungssitze (71) auf der Vorderseite (2G) der Spitze (2F) des Handgriffs (2) ausgebildet sind und das Verriegelungselement (72) während der Bewegung des Knaufs (9) eine Bewegung in Richtung parallel zur Achse (m) des Schafts (6) ausführt.

4. Instrument nach einem der Ansprüche 1 bis 3, **dadurch gekennzeichnet, dass** der Schaft (6) mit einer Ausnehmung (6C) im proximalen Teil des Schaftes (6) versehen ist, die Hülse (5) jedoch mit einem inneren Vorsprung (5G) versehen ist, der in die Ausnehmung (6C) des Schaftes (6) eingeführt wird, um die Position der Hülse (5) in Bezug auf den Schaft (6) zu bestimmen.

5. Instrument nach einem der Ansprüche 2 bis 4, **dadurch gekennzeichnet, dass** die Hülse (5) einen Schlitz (5C) aufweist, der den dem Flansch (5A) gegenüberliegenden Teil der Hülse (5) in zwei Teile (5D, 5E) trennt.

6. Instrument nach einem der Ansprüche 1 bis 5, **dadurch gekennzeichnet, dass** das proximale Ende der Saite (23) eine Verengung (23A) aufweist und mit dem Hebel (3) über ein Gelenk (60) der Saite (23) verbunden ist, das ein zylindrisches Element (61) mit einer geschlitzten Öffnung (63), in der sich ein Einsatz (64) mit einer Kerbe (64A) befindet, gegen die sich die Verengung (23A) der Schnur (23) abstützt, außerdem hat das zylindrische Element (61) eine Queröffnung (62), in der sich das proximale Ende der Schnur (23) befindet.

7. Instrument nach einem der Ansprüche 1 bis 6, **dadurch gekennzeichnet, dass** es mit einem Verriegelungsmechanismus (36) ausgestattet ist, der eine mit dem Hebel (3) verbundene gebogene Zahnstange (37) und einen mit dem Handgriff (2) verbundenen Verriegelungszahn (38) umfasst, der geeignet ist, die Position der Betätigungselemente (24, 35) in der Betriebsposition zu halten, indem die Position des Hebels (3) relativ zum Handgriff (2) gehalten wird, und mit einem Ver- und Entriegelungsmechanismus (40), der mit einem Schalthebel (41) ausgestattet ist, um den Verriegelungsmechanismus (36) in Gang zu halten und um den Verriegelungsmechanismus (36) auszulösen, wobei die gebogene Zahnstange (37) schwenkbar ist, und der Rastzahn (38) unbeweglich am Handgriff (2) angeordnet ist, wobei der am Handgriff (2) schwenkbar gelagerte Schalthebel (41) mit einem Rückschlagelement (43) zum Auslenken der Bogenzahnstange (37) ausgestattet ist, wobei das Rückschlagelement (43) in der Freigabestellung des Schalthebels (41) die Rastmechanik (36) derart außer Eingriff bringt, dass die Bogenzahnstange (37) vom Rastzahn (38) wegbewegt wird, wobei das Rückschlagelement (43) an der Zahnstange (37) des Rastmechanismus (36) anliegt, in der Verriegelungsstellung jedoch das Rückschlagelement (43) von der Bogenzahnstange (37) des Rastmechanismus (36) wegbewegt wird.

8. Instrument nach Anspruch 7, **dadurch gekennzeichnet, dass** der Schalthebel (41) mit Zapfen (41A) zum Einrasten in eine erste Öffnung (47) im Handgriff (2) befestigt ist.

9. Instrument nach einem der Ansprüche 1 bis 8, **dadurch gekennzeichnet, dass** der Hebel (3) mit Zapfen (15) ausgestattet ist, die zur Montage in zweiten Öffnungen (16) im Handgriff (2) geeignet sind.

10. Instrument nach einem der Ansprüche 1 bis 9, **dadurch gekennzeichnet, dass** der Hebel (3) mit einer dritten Öffnung (19) versehen ist, die zur Montage eines Gelenkelements (61) für die Saite (23) geeignet ist.

11. Instrument nach Anspruch 10, **dadurch gekennzeichnet, dass** neben der dritten Öffnung (19) eine vierte Öffnung (19B) zur Befestigung der Kappe (19A) der dritten Öffnung (19) vorhanden ist.

12. Instrument nach einem der Ansprüche 7 bis 11, **dadurch gekennzeichnet, dass** das Pushback-Element (43) als Stift ausgebildet ist.

13. Das Gerät nach einem der Ansprüche
1 bis 12, **dadurch gekennzeichnet, dass** die Aktoren
ausgewählt aus einer Gruppe von Klemmbacken, Fensterbacken, gezahnten Backen, Hakenbacken, Scheren.

14. Vorrichtung nach einem der Ansprüche 1 bis 13, **dadurch gekennzeichnet, dass** der Handgriff (2) mit einem elektrischen Anschluss (12) ausgestattet ist.

## Revendications

1. L'instrument laparoscopique (1, 1') comprenant
une poignée (2);
un levier (3) fixé de manière pivotante à la poignée (2);
une tige (6) encastrée dans un manchon (5) fixé de manière pivotante à la poignée (2), dans laquelle l'extrémité proximale de la tige (6) est encastrée dans le manchon (5), mais où des actionneurs (24, 35) sont fixés à l'extrémité distale de la tige (6) afin d'interagir avec le tissu;
un bouton (9) monté sur le manchon (5) pour faire tourner le manchon (5) avec la tige (6); un mécanisme de verrouillage (70) pour déterminer la position angulaire du manchon (5) et de la tige (6) par rapport à la poignée (2);
une corde (23) pour déplacer les actionneurs (24, 35), dans laquelle l'extrémité distale de la corde (23) est reliée auxdits actionneurs (24, 35), et l'extrémité proximale de la corde (23) est relié au
levier (3), dans lequel la corde (23) est située à l'intérieur de la tige (6), et le mouvement du levier (3) est couplé au mouvement des actionneurs (24, 35), **caractérisé en ce que** le mécanisme de verrouillage (70) est situé à l'extrémité (2F) de la poignée (2) à l'opposé du levier (3), dans lequel l'extrémité (2F) est pourvue de sièges de verrouillage (71), et la partie intérieure (9B) du bouton (9) adjacente au manchon (5) est pourvue d'un élément de verrouillage (72) et d'un ressort (73).

2. L'instrument selon la revendication 1, **caractérisé par le fait que** le manchon (5) a une bride (5A) et une rainure (5B), et la partie intérieure (9B) de forme cylindrique du bouton (9) comporte des fentes (17) séparant la partie intérieure (9B) en segments (14A, 14B, 14C, 14D), dans lesquels au moins un segment (14A, 14C) comporte une butée (14E) orientée vers l'axe (m) de la tige (6), dans lequel au moins une butée (14E) est insérée dans la rainure (5B) pour déterminer la position du bouton (9) par rapport au manchon (5), tandis que la bride (5A) est amenée jusqu'à la surface de retenue (21) dans la poignée (2).

3. L'instrument selon la revendication 1 ou 2, **caractérisé par le fait que** les sièges de verrouillage (71)
sont formés sur la surface avant (2G) de l'extrémité (2F) de la poignée (2), et l'élément de verrouillage (72) effectue un mouvement dans la direction parallèle à l'axe (m) de la tige (6) pendant le mouvement du bouton (9).

4. Instrument selon l'une des revendications 1 à 3, **caractérisé en ce que** la tige (6) est pourvue d'un renfoncement (6C) dans la partie proximale de la tige (6), cependant, le manchon (5) est pourvu d'une protubérance intérieure (5G) qui est insérée dans le renfoncement (6C) de la tige (6) pour déterminer la position du manchon (5) par rapport à la tige (6).

5. L'instrument selon l'une quelconque des revendications 2 à 4, **caractérisé en ce que** le manchon (5) présente une fente (5C) séparant la partie du manchon (5) opposée à la bride (5A) en deux parties (5D, 5E).

6. L'instrument selon l'une quelconque des revendications 1 à 5, **caractérisé en ce que** l'extrémité proximale de la corde (23) présente un rétrécissement (23A) et est reliée au levier (3) au moyen d'une articulation (60) de la corde (23) comprenant un élément cylindrique (61) doté d'une fente l'ouverture (63), dans laquelle se trouve un insert (64) comportant une encoche (64A), contre laquelle s'appuie le rétrécissement (23A) de la corde (23), en outre, l'élément cylindrique (61) présente une ouverture transversale (62) dans laquelle se trouve l'extrémité proximale de la corde (23).

7. L'instrument selon l'une quelconque des revendications 1 à 6, **caractérisé en ce qu'**il est équipé d'un mécanisme de verrouillage (36) comprenant une barre dentée arquée (37) reliée au levier (3) et une dent de verrouillage (38) reliée à la poignée (2), adapté pour retenir la position des actionneurs (24, 35) en position de fonctionnement en maintenant la position du levier (3) par rapport à la poignée (2), et avec un mécanisme de verrouillage et de déverrouillage (40) équipé d'un levier de commutation (41) pour maintenir le mécanisme de verrouillage (36) en prise et pour déclencher le mécanisme de verrouillage (36), dans lequel la barre dentée arquée (37) peut être pivotée, et la dent de verrouillage (38) est positionnée de manière inamovible sur la poignée (2), le levier de commutation (41) monté de manière pivotante sur la poignée (2) étant équipé d'un élément de poussée (43) pour dévier la barre dentée arquée (37), l'élément de poussée (43) en position de relâchement du levier de commutation (41) désengageant le mécanisme de verrouillage (36) de telle manière que que la barre dentée en arc de cercle (37) s'éloigne de la dent de verrouillage (38), l'élément de recul (43) reposant contre la barre dentée (37) du mécanisme de verrouillage (36), alors qu'en position de verrouillage, l'élément de recul (43) s'éloigne de la barre dentée en arc de cercle (37) du mécanisme de verrouillage (36).

8. L'instrument selon la revendication 7, **caractérisé par le fait que** le levier de commutation (41) est fixé avec des pivots (41A) pour s'enclencher dans une première ouverture (47) de la poignée (2).

9. L'instrument selon les revendications 1 à 8, **caractérisé par le fait que** le levier (3) est équipé de pivots (15), qui sont adaptés pour être montés dans les deuxièmes ouvertures (16) de la poignée (2).

10. L'instrument selon les revendications 1 à 9, **caractérisé par le fait que** le levier (3) est pourvu d'une troisième ouverture (19), adaptée au montage d'un élément d'articulation (61) pour la corde (23).

11. L'instrument selon la revendication 10, **caractérisé en ce que**, à côté de la troisième ouverture (19), il y a une quatrième ouverture (19B) pour fixer le capuchon (19A) de la troisième ouverture (19).

12. L'instrument selon l'une quelconque des revendications 7 à 11, **caractérisé par le fait que** l'élément de poussée (43) est constitué d'une goupille.

13. L'instrument selon les revendications
1 à 12, **caractérisés par le fait que** les actionneurs sont
choisi dans un groupe comprenant les mâchoires de serrage, les mâchoires à fenêtre, les mâchoires dentées, les mâchoires à crochet, les ciseaux.

14. Le dispositif ac selon les revendications 1 à 13, **caractérisé par le fait que** la poignée (2) est équipée d'un connecteur électrique (12).
